# EUROPEAN PATENT SPECIFICATION

(11) **EP 1 502 957 B1**
(45) Date of publication and mention of the grant of the patent: **27.08.2008**
(21) Application number: 04016242.2
(22) Date of filing: 09.07.2004
(51) Int. Cl.: C12Q 1/00, G01N 33/66

(54) **Castable diffusion membrane for enzyme-based sensor application**
Giessbarer Diffusionsmembran für enzym-basierte Sensoren
Membrane de diffusion à couler pour des capteur basés sur un enzyme

(30) Priority: 11.07.2003 EP 03015882
(43) Date of publication of application: 02.02.2005
(73) Proprietor: F. Hoffmann-La Roche AG, 4070 Basel (CH); Roche Diagnostics GmbH, 68305 Mannheim (DE)
(72) Inventor: Desprez, Valerie, 69115 Heidelberg (DE)
(74) Representative: Dey, Michael

(56) References cited:
- EP-A- 0 175 195
- WO-A-01/69222
- WO-A-99/30152
- US-A- 4 900 933
- US-A- 5 124 128
- US-A- 5 422 246
- US-A- 5 633 081
- US-A- 6 107 083
- US-A- 6 156 550
- US-B1- 6 214 185

## Description

The invention relates to a diffusion membrane for an enzyme-based sensor, a sensor comprising said diffusion membrane as well as a method for producing said enzyme-based sensor and the use of said enzyme-based sensor for the detection and/or determination of a substance, in particular an enzyme substrate, e.g. glucose.

Enzyme-based sensors are widely used to determine substances of interest in qualitative as well as quantitative manner in the blood and in other body liquids. Enzyme-based sensors are in particlular used for the determination of enzyme substrates. In an enzyme-based sensor a so-called sensing reaction (sometimes also refered to in the art as "transducer reaction") occurs wherein a substance is converted under participation of at least one enzyme into another substance, which can be detected directly or indirectly. An example of such a sensing reaction is the enzyme catalyzed oxidation of glucose. Usually, this reaction uses oxygen as electron acceptor. In the course of the reaction, glucose is converted into gluconolactone and the oxygen is converted into hydrogen peroxide. A sensing reaction either could measure the consumption of glucose and oxygen or the production of hydrogen peroxide or gluconolactone.

An enzyme-based sensor usually comprises several layers, among them an enzyme layer and a cover membrane or outer layer. This cover membrane is directly in contact with the sample and limits the diffusion of the substances necessary for the sensing reaction, especially the enzyme substrate or cosubstrate.

Enzyme-based sensors can be provided as electrochemical sensors or as optical sensors (optodes). The construction and function of a glucose optode is for example described in U.S. 6,107,083. The construction and function of an electrochemical glucose sensor is for example described in WO 99/30152.

Particularly, enzyme-based sensors which are used for the determination of glucose, lactate or creatinine are preferably constructed with oxidoreductases and the detection is based on the oxygen consumption. In this case, the sensor necessits a cover membrane being a porous or at least a permeable polymer membrane, which controls the permeation of both the enzyme substrate and oxygen.

The currently available diffusion membranes for enzyme-based sensor application suffer from various disadvantages. According to one approach known in the state of the art cover membranes for enzyme-based sensor applications are preformed membranes consisting of microporous structures from non-hydrating polymers like polycarbonate, polypropylene and polyesters. The porosity of such membranes is provided by physical means, e.g. by neutron or argon track etching. Glucose and oxygen permeate across such membranes predominantly in these pores filled with blood or other body liquids. One major disadvantage is that such membranes are preformed and not castable. A preformed membrane has to be attached to the enzyme layer. Very often the membranes are mechanically attached to the enzyme layer. Such mechanical attachments are expensive and technically complex. Further problems occur insofar as it is difficult to apply the membrane onto the underlaying layer without producing air bubbles. Similar problems also occur when the membrane is for example glued onto an underlaying layer.

Another approach known in the state of the art are castable cover membranes. Such cover membranes are generally formed by applying a solution of a polymer to an enzyme layer and by evaporating the solvent. Such membranes consist of polymer structures with hydrophilic and hydrophobic regions. Upon exposure to water, the hydrophilic region of the membranes absorb water, thus providing in the swelled structure a permeation path e.g. for glucose. However, those membranes provide no defined porosity. In this approach the polymer itself has to provide the permeation, therefore not all polymers are suitable and thus the election of polymers is limited.

One disadvantage is that polymers, which are suitable for the use of castable membranes, are very often soluble only in aggressive or toxic solvents. Examples for this are cellulose acetate, which is soluble in DMSO and acetone, and PVC, which is soluble in tetrahydrofurane and cyclohexanone. This circumstance is relevant not only for safety reasons but also because the enzymes present in the enzyme layer may be destroyed by these solvents. Moreover, the effectiveness of such a membrane depends upon the dispersion of the hydrophilic domains within the hydrophobic matrix. Since it is difficult to achieve a homogenous dispersion in and during the production process of the membrane, the consequence is an inhomogenous distribution of the hydrophilic domains. This results for example in a poor reproducibility.

Offenbacher et al. (U.S. 6,214,185) describe a cover membrane with better coating reproducibility. Said membrane is made of a PVC copolymer which allows a quite satisfying adjustment of the permeability due to the presence of a hydrophilic copolymer component. However, such a PVC cover membrane shows limitations for multiple measurements when used for a sensor based on the consumption of oxygen since the regeneration of the oxygen reservoir of the sensor is very slow.

US-A-5,124,128 discloses a process for producing a porous membrane in which two immiscible polymers are mixed with filler particles.

It is an object of the present invention to overcome at least partially the described drawbacks of the membranes for enzyme-based sensor application. In particular, it is an object to provide a castable membrane with a good processability. A further object is the provision of a membrane with a defined permeability, which can also be used for multiple measurements.

The present invention therefore relates to a diffusion membrane for an enzyme-based sensor application, wherein the membrane comprises
(a) at least one polymer material and
(b) pore-maker particles forming continuous pores dispersed in the at least one polymer material.

The membrane according to the invention comprises pore-maker particles dispersed in one or more polymer materials. The porosity of the membrane is provided by the pore-maker particles. Thus, from the permeability or porosity point of view there is no limitation when electing the polymer.

The polymer material used for the diffusion membrane of the invention can generally be any castable polymer material or a mixture of polymer materials. According to the planned application, for example non-toxic or easily applicable materials can be used.

Preferred polymer materials are selected from the group consisting of non-water soluble polymers and preferably from polyurethane, polyacrylamide, polystyrene, polyvinyl esters and co-polymers of, e.g., butadiene and styrene.

Due to the various election possibilities with regard to the polymer material, a castable diffusion membrane can be provided easily, which can be coated directly in a reproducible manner. The diffusion membrane can for example be coated on an underlaying layer, preferably onto an enzyme layer. It is an advantage of the diffusion membrane of the present invention that directly coating the diffusion membrane onto the enzyme layer does not alter the related enzyme activity. Furthermore, a diffusion membrane can easily be provided, which is insoluble in body liquids such as for example blood.

The diffusion membrane according to the invention comprises pore-maker particles dispersed in the layer forming polymer material. The pore-maker particles provide the porosity of the membranes and thus the permeability. The particles used as pore-maker are preferably stable particles or mixtures of such particles possessing inherent and defined porosity. The size of the pore-maker particles is preferably between about 0.5-100 µm, particularly from about 1 to 50 µm.

The membrane has a defined porosity, which is provided by pores, formed by the pore-maker particles according to the invention. According to the application of the membrane, the size of the pores can be varied. The size of the pores is preferably within the range of the size of the pore-maker particles.

The diffusion membrane according to the invention comprises pore-maker particles in an amount of preferably about 0.5-70 weight %, more preferably from about 0.7-50 weight %, most preferably from about 1-40 weight % based on the total weight of the dried membrane. If the proportion of the pore-maker particles exceeds a certain limit, then the membrane becomes mechanically instable; if too little pore-maker particles are added then the membrane could become impermeable.

For the use as pore-maker particles in the membrane essentially all stable particles and mixtures of such particles are useful, which possess an inherent and defined porosity. According to the desired application and/or pore size suitable particles can be elected. Examples for suitable pore-maker particles include inorganic or organic particles made from Kieselguhr, silica gel, cellulose, precipitated gypsium, kaolin, glass or the like. Particularly preferred particles are based on silicon dioxide, and more especially on diatomaceous earth. An especially preferred diatomeous earth is sold under the tradename Celatom®.

The diffusion membrane according to the invention can further comprise other elements such as for example carbon black for optical isolation, pigments like titanium dioxide for improved remission properties of the membrane, or wetting agents.

The thickness of the diffusion membrane according to the invention can be chosen flexibly with regard to the desired use and/or permeation rate. Suitable thicknesses are within the range of about 0,5-1000 µm, preferably about 3-500 µm, most preferably 5-100 µm.

The permeation of the diffusion membrane can thus be easily adjusted by varying the coating thickness and/or the concentration of the pore-maker particles.

In one embodiment of the diffusion membrane according to the invention, the size of the pore-maker particles corresponds at least to the thickness of the layer of the diffusion membrane, the relation between the particle size and the layer thickness is approximately 1:1 or ≥ 1:1. The size of the pore-maker particles is chosen in a way that the single particles or clusters of single particles form continous pores from the surface of the sensor to the enzyme layer. Thus, a diffusion of the substances is provided.

A further aspect of the invention is an enzyme-based sensor comprising a diffusion membrane as described above. A sensor according to the invention comprises several layers, wherefrom at least one layer is an enzyme layer. The enzyme-based sensor of the invention further comprises a cover layer and at least one underlaying layer. Depending on the type of the sensor, further layers can for example be an interference-blocking layer, a layer for optical isolation, an electro-conductive layer, an indicator layer or a base electrode.

Since the permeability of the diffusion membrane can be adjusted as desired, the diffusion membrane provides a fast regeneration of the sensor. In the case of a sensing reaction based for example on the consumption of oxygen, the oxygen permeation can be adjusted in such a manner that the sensor regeneration, e.g. the regeneration of the oxygen reservoir is very fast. Thus, the sensor of the invention can also be used for multiple measurements.

The enzyme layer of the enzyme-based sensor can for example comprise oxidative enzymes as for example glucose oxidase, cholesterol oxidase or lactate oxidase. The enzyme layer may also comprise an enzyme mixture, such as for example an enzyme cascade, which makes possible the detection of analytes which cannot be directly detected, such as for example the creatine. Creatine cannot be enzymatically oxidized by a simple enzyme but requires several enzymatic steps to generate an analyte derivative, which is detectable by optical or amperometric means. A suitable enzyme cascade system for the detection and/or determination of creatinin, comprises e.g. creatinine amidohydrase, creatinine amidohydrolase, and sarcosine oxidase.

In the sensor according to the invention, the diffusion membrane is preferably deposited as cover layer. In this case, after solvent evaporation of the dispersion a stable cover layer is formed. The diffusion membrane is further preferably coated directly on an underlaying layer, preferably an enzyme layer. By a direct coating of the membrane, favourably, the membrane layer is attached to the underlaying layer by physical adhesion without a mechanical fixation and/or the use of glue.

When the diffusion membrane is used as cover layer, it is directly in contact with the test sample and regulates the diffusion of the substances necessary for the sensing reaction, preferably the substrates or cosubstrates. The diffusion membrane according to the invention provides thus the sensor with a controlled permeability.

The enzyme-based sensor of the invention can basically represent any kind of a biosensor. Examples for suitable biosensors are for example optical sensors. With preferred optical sensors, the consumption of oxygen due to an enzymatic reaction can be detected using an appropriate dye which is sensitive to oxygen, e.g., a luminescent dye quenchable with oxygen. Furthermore, an electrochemical sensor is suitable for the use in the present invention.

Especially in connection with sensors which use oxygen consumption as a means for analyte determination, the membranes according to this invention show big advantages. The pores created by the pore-maker particles allow for the adjustment of diffusion of the analyte molecules, e.g., glucose, across the membrane, and the choice of the polymer influences the permeability of oxygen. Although oxygen permeation across the membrane in part is also possible through the pores created by the pore-maker particles, it essentially is influenced by the polymer layer, especially if this has a high oxygen permeability.

A further aspect of the present invention is the use of an enzyme-based sensor as described above for the detection or quantitative determination of a substance, preferably an enzyme substrate.

In the field of medicine, a possibility of the use is for example the determination of physiological parameters. A determination and/or detection can be carried out in any liquid, for example in various body liquids such as blood, serum, plasma, urine, and the like. A preferred use of the sensor is a detection and/or determination of analytes in blood.

A possible use of the sensors according to the invention is for example the determination of blood glucose in patients suffering from diabetes. Other metabolic products that can be determined with the enzyme-based sensor according to the invention are for example cholesterol or urea.

Another possible use of the enzyme-based sensor of the invention is in the field of environmental analytic, process control in biotechnology and food control.

With the use according to the invention of the enzyme-based sensor a wide variety of substances, for example enzyme substrates and/or cosubstrates can be determined and/or detected. Suitable enzyme substrates are for example cholesterol, succrose, glutamate, ethanole, ascorbic acid, fructose, pyruvat, ammonium, nitrite, nitrate, phenol, NADH, glucose, lactate or creatinine. Preferably, a determination and/or detection of glucose, lactate or creatinine is performed. A particularly preferred substance to be detected and/or determined is glucose.

Since the regeneration of the enzyme-based sensor can be influenced by adjusting the permeation, the regeneration is fast enough to allow multiple measurements. In a preferred use of the sensor multiple measurements are performed. Further, the enzyme-based sensor can be employed for every sensor-application known in the art, such as for example for a single use application or as a permanent sensor for multi use applications.

A further subject matter of the present invention is a method for the preparation of an enzyme-based sensor as described above. This method comprises:
(i) forming a dispersion comprising
   (a) at least one polymer material and
   (b) pore-maker particles
(ii) casting the dispersion directly on an underlaying layer to form a diffusion membrane and
(iii) optionally drying the dispersion.

The method according to the invention allows a direct casting of the membrane due to the broad option of polymer materials. By using suitable polymer materials, it is furthermore possible to work without solvents, if desired. Further, the materials can be elected in a way that a heating of the dispersion is not necessary. Thus, by the method according to the invention, an easy handling is provided.

The method allows the application of the membrane without damaging lower layers, e.g. the enzyme layer. The membrane can for example be applied directly onto the enzyme layer without influencing the enzymes. In a preferred embodiment, the dispersion is casted directly on an enzyme layer.

In the method according to the invention, the dispersion is preferably attached to the underlaying layer by physical adhesion. Thus, a mechanical attachment of the membrane including the above mentioned disadvantages is not necessary.

If desired, the dispersion can be dried after the application on the underlaying layer. Essentially, every drying method known in the technical field can be used.

The following examples 1 and 2 illustrate the invention:

### Example 1

Membrane for the determination of the glucose concentration in liquid samples.

For the preparation of the membrane dispersion, the following components were mixed:

**Table 1**

| | |
|---|---|
| Polyurethane dispersion (20% in ethanole) (Tyndale Plains-Hunter, Ltd.) | 2g |
| Celatome MW 27 (Eagle-Picher Minerals, Inc.) | 0,2g |
| optionally Carbon black | 0,06g |

The mixture was applied on top of the enzyme layer of an OptiCCA single use glucose sensor (Roche Diagnostics Corp.) in a 20 µm thick layer. After drying at room temperature, a sensor spot (4mm diameter) overcoated with the membrane of the invention was cut from the sensor foil and placed in a flow through cell of which the channel was filled with an appropriate buffer before injecting the sample.

Raw intensities produced by the luminescence quenching of the luminescent dye contained in the oxygen sensitive layer were then measured. The following kinetic measurements (fluorescence intensity change: ΔI per second) were obtained with three different glucose control solutions tonometered at 150 Torr oxygen partial pressure.

**Table 2**

| **Glucose concentration** **(mg/dL)** | **Relative slope** (ΔI per second) |
|---|---|
| 50 | 565 |
| 113 | 3972 |
| 356 | 28796 |

### Example 2

Example 2 shows the fast sensor regenerations and the possibilty of multiple measurements (3 measurements with the control solution containing 356 mg/dL glucose and 150 Torr oxygen partial pressure). The sensor used in Example 2 was prepared according to Example 1, but was not completely identical to the sensor of Example 1 due to handcoating of the membrane.

**Table 3**

| **No. of measurements** | **Relative slope** (ΔI per second) | **Regeneration time** (seconds) |
|---|---|---|
| 1 | 34439 | <60 |
| 2 | 36906 | <60 |
| 3 | 35760 | <60 |
| **CV%** | **3,5** | |

## Claims

1. Diffusion membrane for an enzyme-based sensor application, wherein the membrane comprises
(a) at least one polymer material and
(b) pore-maker particles forming continuous pores dispersed in the at least one polymer material.

2. Diffusion membrane according to claim 1, wherein the size of the pore-maker particles is from 0,5-100 *µ*m.

3. Diffusion membrane according to claim 1 or 2, wherein the pore-maker particles are present in an amount of 0,5-70% by weight.

4. Diffusion membrane according to any of claims 1-3, wherein the pore-maker particles comprise diatomaceous earth.

5. Diffusion membrane according to any of claims 1-4, wherein the thickness of the membrane is 0,5-1000 *µ*m.

6. Enzyme-based sensor comprising a diffusion membrane as claimed in any of claims 1-5.

7. Enzyme-based sensor according to claim 6, comprising
(i) at least one enzyme layer
(ii) a cover layer and
(iii) at least one underlaying layer.

8. Enzyme-based sensor according to claims 6 or 7, wherein the diffusion membrane is the cover layer.

9. Enzyme-based sensor according to any of claims 6 to 8, wherein the diffusion membrane is coated directly on an underlaying layer.

10. Enzyme-based sensor according to any of claims 6-9, wherein the diffusion membrane is attached to the underlaying layer by physical adhesion.

11. Enzyme-based sensor according to any of claims 6-8, wherein the diffusion membrane is coated directly on an enzyme layer.

12. Enzyme-based sensor according to any of claims 6-11, wherein the sensor is an optical sensor.

13. Enzyme-based sensor according to any of claims 6-11, wherein the sensor is an electrochemical sensor.

14. Use of an enzyme-based sensor according to any of claims 6-13 for the detection and/or quantitative determination of an enzyme substrate and/or cosubstrate.

15. Use according to claim 14, wherein the enzyme substrate is glucose.

16. Use according to claim 14 or 15, wherein the determination is performed in blood.

17. Use according to any of claims 14-16, wherein multiple measurements are performed.

18. Method for the preparation of an enzyme-based sensor, comprising
(i) forming a dispersion comprising
(a) at least one polymer material and
(b) pore-maker particles, forming continuous pores,
(ii) casting the dispersion directly on an enzyme layer to form a diffusion membrane and
(iii) optionally drying the dispersion.

19. Method according to claim 18, wherein the dispersion is attached to the underlaying layer by physical adhesion.

## Patentansprüche

1. Diffusionsmembran für eine Enzym-basierte Sensoranwendung, wobei die Membran umfasst
(a) mindestens ein polymeres Material und
(b) Poren-machende Partikel, die kontinuierliche Poren bilden, die in dem mindestens einem polymeren Material dispergiert sind.

2. Diffusionsmembran gemäß Anspruch 1, wobei die Größe der Poren-machenden Partikel von 0,5-100 µm liegt.

3. Diffusionsmembran gemäß Anspruch 1 oder 2, wobei die Poren-machenden Partikel in einer Menge von 0,5-70 Gew.-% vorhanden sind.

4. Diffusionsmembran gemäß einem der Ansprüche 1-3, wobei die Poren-machenden Partikel Kieselgur enthalten.

5. Diffusionsmembran gemäß einem der Ansprüche 1-4, wobei die Dicke der Membran 0,5-1000 µm ist.

6. Enzym-basierter Sensor, umfassend eine Diffusionsmembran gemäß einem der Ansprüche 1-5.

7. Enzym-basierter Sensor gemäß Anspruch 6, umfassend
(i) mindestens eine Enzymschicht,
(ii) eine Deckschicht und
(iii) mindestens eine darunter liegende Schicht.

8. Enzym-basierter Sensor gemäß den Ansprüchen 6 oder 7, wobei die Diffusionsmembran die Deckschicht ist.

9. Enzym-basierter Sensor gemäß einem der Ansprüche 6-8, wobei die Diffusionsmembran direkt auf eine darunter liegende Schicht aufgebracht ist.

10. Enzym-basierter Sensor gemäß einer der Ansprüche 6-9, wobei die Diffusionsmembran an die darunter-liegende Schicht durch physikalische Adhäsion angebracht ist.

11. Enzym-basierter Sensor gemäß einer der Ansprüche 6-8, wobei die Diffusionsmembran direkt auf einer Enzymschicht aufgebracht ist.

12. Enzym-basierter Sensor gemäß einer der Ansprüche 6-11, wobei der Sensor ein optischer Sensor ist.

13. Enzym-basierter Sensor gemäß einer der Ansprüche 6-11, wobei der Sensor ein elektrochemischer Sensor ist.

14. Verwendung eines Enzym-basierten Sensors gemäß einer der Ansprüche 6-13 zum Nachweis und/oder zur quantitativen Bestimmung eines Enzymsubstrats und/oder Cosubstrats.

15. Verwendung gemäß Anspruch 14, wobei das Enzymsubstrat Glucose ist.

16. Verwendung gemäß Anspruch 14 oder 15, wobei die Bestimmung in Blut durchgeführt wird.

17. Verwendung gemäß einem der Ansprüche 14-16, wobei viele Messungen durchgeführt werden.

18. Verfahren zur Herstellung eines Enzym-basierten Sensors, umfassend
(i) Bilden einer Dispersion, umfassend
(a) mindestens ein polymeres Material und
(b) Poren-machende Partikel, die kontinuierliche Poren bilden,
(ii) Gießen der Dispersion direkt auf eine Enzymschicht, um eine Diffusionsmembran zu bilden und
(iii) gegebenenfalls Trocknen der Dispersion.

19. Verfahren gemäß Anspruch 18, wobei die Dispersion auf die unterliegende Schicht durch physikalische Adhäsion angebracht ist.

## Revendications

1. Membrane de diffusion pour une application de capteur à base d'enzymes, la membrane comprenant:
(a) au moins un matériau polymère et
(b) des particules porogènes formant des pores continus dispersées dans l'au moins un matériau polymère.

2. Membrane de diffusion selon la revendication 1, dans lequel la taille des particules porogènes est de 0,5-100 µm.

3. Membrane de diffusion selon la revendication 1 ou 2, dans laquelle les particules porogènes sont présentes en une quantité de 0,5-70 % en masse.

4. Membrane de diffusion selon l'une quelconque des revendications 1-3, dans laquelle les particules porogènes comprennent de la terre à diatomées.

5. Membrane de diffusion selon l'une quelconque des revendications 1-4, l'épaisseur de la membrane étant de 0,5-1000 µm.

6. Capteur à base d'enzymes comprenant une membrane de diffusion selon l'une quelconque des revendications 1-5.

7. Capteur à base d'enzymes selon la revendication 6, comprenant:
(i) au moins une couche d'enzyme
(ii) une couche de couverture et
(iii) au moins une couche sous-jacente.

8. Capteur à base d'enzymes selon les revendications 6 ou 7, dans lequel la membrane de diffusion est la couche de couverture.

9. Capteur à base d'enzymes selon l'une quelconque des revendications 6 à 8, dans lequel la membrane de diffusion est appliquée directement sur une couche sous-jacente.

10. Capteur à base d'enzymes selon l'une quelconque des revendications 6-9, dans lequel la membrane de diffusion est fixée sur la couche sous-jacente par une adhésion physique.

11. Capteur à base d'enzymes selon l'une quelconque des revendications 6-8, dans lequel la membrane de diffusion est appliquée directement sur une couche d'enzyme.

12. Capteur à base d'enzymes selon l'une quelconque des revendications 6-11, le capteur étant un capteur optique.

13. Capteur à base d'enzymes 6-11, le capteur étant un capteur électrochimique.

14. Utilisation d'un capteur à base d'enzymes selon l'une quelconque des revendications 6-13 pour la détection et/ou la détermination quantitative d'un substrat et/ou d'un cosubstrat d'enzyme.

15. Utilisation selon la revendication 14, dans laquelle le substrat d'enzyme est le glucose.

16. Utilisation selon la revendication 14 ou 15, dans laquelle la détermination s'effectue sur du sang.

17. Utilisation selon l'une quelconque des revendications 14-16, dans laquelle on effectue des mesures multiples.

18. Procédé de préparation d'un capteur à base d'enzymes, comprenant:
(i) la formation d'une dispersion comprenant
(a) au moins un matériau polymère et
(b) des particules porogènes formant des pores continus,
(ii) la coulée de la dispersion directement sur une couche d'enzyme pour former une membrane de diffusion et
(iii) éventuellement le séchage de la dispersion.

19. Procédé selon la revendication 18, dans lequel la dispersion est fixée à la couche sous-jacente par adhésion physique.
